# EUROPÄISCHE PATENTSCHRIFT

(11) **EP 3 049 186 B1**
(45) Veröffentlichungstag und Bekanntmachung des Hinweises auf die Patenterteilung: **15.11.2017**
(21) Anmeldenummer: 14772105.4
(22) Anmeldetag: 19.09.2014
(51) Int. Cl.: B01L 3/00, B01L 7/00

(54) **ANALYSEEINHEIT ZUM DURCHFÜHREN EINER POLYMERASEKETTENREAKTION, VERFAHREN ZUM BETREIBEN EINER SOLCHEN ANALYSEEINHEIT UND VERFAHREN ZUM HERSTELLEN EINER SOLCHEN ANALYSEEINHEIT**
ANALYTICAL DEVICE FOR PERFORMING PCR, METHOD FOR OPERATING THE DEVICE AND METHOD OF MAKING THE DEVICE
DISPOSITIF D'ANALYSE , PROCÉDÉ DE FONCTIONNEMENT DU DISPOSITIF ET PROCÉDÉ DE SA FABRICATION

(30) Priorität: 27.09.2013 DE 102013219502
(43) Veröffentlichungstag der Anmeldung: 03.08.2016
(73) Patentinhaber: Robert Bosch GmbH, 70442 Stuttgart (DE)
(72) Erfinder: BRETTSCHNEIDER, Thomas, 71229 Leonberg (DE); HOFFMANN, Jochen, 71229 Leonberg (DE)
(86) Internationale Anmeldenummer: PCT/EP2014/069963
(87) Internationale Veröffentlichungsnummer: WO 2015/044040

(56) Entgegenhaltungen:
- US-A1- 2005 180 891
- US-A1- 2006 275 852

## Beschreibung

### Stand der Technik

Die vorliegende Erfindung bezieht sich auf eine Analyseeinheit zum Durchführen einer Polymerasekettenreaktion, auf ein Verfahren zum Betreiben einer solchen Analyseeinheit und auf ein Verfahren zum Herstellen einer solchen Analyseeinheit.

Die digitale Polymerasekettenreaktion ermöglicht den Nachweis einzelner DNA-Sequenzen in einem Kavitätenarray. Hierfür werden n DNA-Moleküle zusammen mit einem biochemischen Amplifikationssystem (Polymerase Chain Reaction; PCR) auf m Mikrokavitäten eines Chips statistisch verteilt, wobei im Normalfall n kleiner m ist. In jeder Kavität, in der initial ein DNA-Molekül war, werden Millionen identischer Kopien des DNA-Moleküls und gleichzeitig ein detektierbares Signal generiert. Durch Auszählen der Signale kann somit nach der Polymerasekettenreaktion zwischen leeren (= 0) und Kavitäten mit einem DNA-Molekül (= 1) "digital" unterschieden werden.

In kommerziell erhältlichen Systemen für digitale Polymerasekettenreaktionen wird eine biologische Probe zunächst aufgeschlossen, dann die DNA aufgereinigt und extrahiert.

Mikrofluidische Diagnosesysteme wie Lab-on-a-Chip (LOC; "Chiplabor") erlauben die miniaturisierte und integrierte Durchführung komplexer Arbeitsabläufe wie zum Beispiel eine voll automatisierte Probenvorbereitung. In solchen Lab-on-a-Chip-Systemen kann zunächst das zu untersuchende Probenmaterial mittels Polymerasekettenreaktion amplifiziert und anschließend auf einem Mikroarray analysiert werden. Die Verknüpfung beider Operationen und die mehrschrittige Prozessführung bedingen eine verlängerte Prozesszeit und eine aufwendige Prozessführung. Die digitale Polymerasekettenreaktion hingegen erlaubt es, DNA-Moleküle spezifisch in einem Schritt zu amplifizieren und zu quantifizieren.

### Offenbarung der Erfindung

Vor diesem Hintergrund werden mit der vorliegenden Erfindung eine Analyseeinheit zum Durchführen einer Polymerasekettenreaktion, ein Verfahren zum Betreiben einer solchen Analyseeinheit und ein Verfahren zum Herstellen einer solchen Analyseeinheit gemäß den Hauptansprüchen vorgestellt. Vorteilhafte Ausgestaltungen ergeben sich aus den jeweiligen Unteransprüchen und der nachfolgenden Beschreibung.

Es wird eine Analyseeinheit zum Durchführen einer Polymerasekettenreaktion vorgestellt, wobei die Analyseeinheit folgende Merkmale aufweist:
ein Deckelelement;
ein Bodenelement mit zumindest einer Bodenausnehmung, wobei die Bodenausnehmung eine Fluidaufnahmefläche und eine Anordnung aus Mikrokavitäten aufweist, wobei die Bodenausnehmung dem Deckelelement gegenüberliegend angeordnet ist;
zumindest einen Fluidkanal, der zwischen dem Deckelelement und dem Bodenelement ausgebildet ist, um ein Fluid auf die Fluidaufnahmefläche der Bodenausnehmung zu leiten;
zumindest einen Druckkanal, der in dem Deckelelement ausgebildet ist, um einen Druck in einen Bereich der Fluidaufnahmefläche zu leiten; und
eine Folie, die im Bereich der Bodenausnehmung zwischen dem Deckelelement und dem Bodenelement angeordnet ist, wobei die Folie ausgebildet ist, um bei Leiten des Drucks in den Bereich der Fluidaufnahmefläche durch den Druck derart verformt zu werden, dass das Fluid von der Fluidaufnahmefläche in die Anordnung aus Mikrokavitäten bewegt wird.

Unter einem Deckelelement und einem Bodenelement kann je eine Schicht verstanden werden, die beispielsweise aus einem Kunststoff, insbesondere aus einem Polymer, gefertigt ist. Unter einer Bodenausnehmung kann beispielsweise eine Vertiefung in dem Bodenelement verstanden werden. Unter einer Fluidaufnahmefläche kann eine Fläche der Bodenausnehmung verstanden werden, auf die ein Fluid aufgebracht werden kann. Unter einem Fluid kann eine Flüssigkeit mit biochemischem Material wie etwa Nukleinsäuren verstanden werden. Unter einer Mikrokavität kann eine in der Bodenausnehmung ausgebildete Vertiefung zum Aufnehmen des Fluids verstanden werden. Beispielsweise kann das Fluid in die Mikrokavität eingebracht werden, um eine Polymerasekettenreaktion durchzuführen. Unter einer Folie kann ein flächiges flexibles Element wie beispielsweise eine Kunststofflage verstanden werden. Die Folie kann dabei beispielsweise fluidundurchlässig sein.

Der vorliegende Ansatz beruht auf der Erkenntnis, dass es möglich ist, ein Array aus Mikrokavitäten zum Durchführen von Analysen mittels Polymerasekettenreaktion in ein Lab-on-a-chip-System zu integrieren. In dem Lab-on-a-chip-System kann beispielsweise auch eine Vorbereitung einer zu analysierenden Probenlösung stattfinden. Vorteilhafterweise kann eine Membran des Lab-on-a-chip-Systems verwendet werden, um die Probenlösung nach der Vorbereitung in die Mikrokavitäten zu füllen. Hierzu kann die Membran mittels eines Drucks derart ausgelenkt werden, dass die Probenlösung in die Mikrokavitäten gedrückt wird.

Dadurch, dass das Mikroarray automatisch und kontrolliert befüllt werden kann, kann ein Prozessablauf im Vergleich zu einem manuellen Transferieren der Probenlösung deutlich beschleunigt und vereinfacht werden. Ferner können dadurch Bedienungsfehler ausgeschlossen werden und Kosten für speziell trainiertes Personal gespart werden. Schließlich bietet der vorliegende Ansatz den Vorteil einer verringerten Kontaminationsgefahr.

Gemäß einer Ausführungsform des vorliegenden Ansatzes kann der Druckkanal als Durchgangsöffnung in dem Deckelelement ausgebildet sein. Hierbei kann die Bodenausnehmung derart angeordnet sein, dass die Fluidaufnahmefläche der Durchgangsöffnung gegenüberliegt und die Anordnung aus Mikrokavitäten seitlich zur Durchgangsöffnung versetzt ist. Dadurch kann mit geringem Kostenaufwand eine effiziente Ansteuerung der Folie realisiert werden.

Gemäß einer Ausführungsform des vorliegenden Ansatzes kann die Folie zumindest im Bereich der Fluidaufnahmefläche und/oder im Bereich der Anordnung aus Mikrokavitäten lösbar an dem Deckelelement befestigt sein. Dadurch kann erreicht werden, dass sich die Folie bei Anlegen des Drucks an der Durchgangsöffnung kontinuierlich von der Fluidaufnahmefläche in Richtung der Mikrokavitäten auswölbt. Dies bietet den Vorteil einer sehr zuverlässigen Befüllung der Mikrokavitäten.

Ferner kann die Folie ausgebildet sein, um durch den Druck derart verformt zu werden, dass die Anordnung aus Mikrokavitäten fluiddicht verschlossen wird. Dadurch, dass die Mikrokavitäten in einem Schritt befüllt und fluiddicht verschlossen werden, kann eine frühzeitige Verdunstung des Fluids verhindert werden.

Zwischen der Anordnung aus Mikrokavitäten und der Fluidaufnahmefläche kann zudem ein Trennelement ausgebildet sein, um ein Bewegen des Fluids von der Fluidaufnahmefläche in die Anordnung aus Mikrokavitäten vor Leiten des Drucks in den Bereich der Fluidaufnahmefläche zu verhindern. Unter einem Trennelement kann beispielsweise eine quer zu einer Bewegungsrichtung des Fluids verlaufende Rille in der Bodenausnehmung oder ein auf die Bodenausnehmung aufgebrachter Streifen aus einem hydrophoben Material verstanden werden. Mittels eines solchen einfach zu realisierenden Trennelements kann das Fluid schnell und kontrolliert in die Analyseeinheit eingebracht werden.

Gemäß einer weiteren Ausführungsform des vorliegenden Ansatzes kann die Fluidaufnahmefläche zumindest teilweise mit einer komprimierbaren Faserschicht zum Aufnehmen und/oder Abgeben des Fluids bedeckt sein. Unter einer Faserschicht kann eine Schicht aus einem flüssigkeitsabsorbierenden Material wie beispielsweise Vlies verstanden werden. Beispielsweise kann die Faserschicht ausgebildet sein, um ein vorbestimmtes Maximalvolumen des Fluids zu absorbieren. Somit kann nicht nur ein unbeabsichtigtes Bewegen des Fluids von der Fluidaufnahmefläche in die Anordnung aus Mikrokavitäten verhindert werden, sondern auch eine Menge des Fluids abgemessen werden.

Des Weiteren kann das Bodenelement im Bereich der Anordnung aus Mikrokavitäten eine vorbestimmte Maximaldicke aufweisen. Beispielsweise bietet eine geringe Maximaldicke den Vorteil, dass die Mikrokavitäten zum Durchführen einer temperaturgesteuerten Polymerasekettenreaktion schnell und effizient temperiert werden können. Ferner kann eine Lichtabsorption durch das Bodenelement im Bereich der Mikrokavitäten reduziert werden. Dies kann insbesondere beim Anwenden optischer Verfahren zum Analysieren des Fluids von Vorteil sein.

Die Folie kann zumindest im Bereich der Anordnung aus Mikrokavitäten eine Isolierschicht aufweisen und/oder als Mehrschichtverbund ausgebildet sein, um eine Dampfdurchlässigkeit der Folie zu verringern. Dadurch kann Diffusionsverlusten effizient entgegengewirkt werden und eine hohe Zuverlässigkeit der Analyseeinheit gewährleistet werden.

Die Isolierschicht kann gemäß einer weiteren Ausführungsform auf einer dem Deckelelement zugewandten Seite der Folie ausgebildet sein. Insbesondere kann hierbei die Isolierschicht aus Paraffin gebildet sein. Indem das Paraffin einen festen Aggregatszustand aufweist und beispielsweise erst dann in einen flüssigen Aggregatszustand wechselt, wenn die Analyseeinheit erwärmt wird, kann die Isolierschicht bei der Herstellung der Analyseeinheit sehr einfach auf die Folie aufgebracht werden.

Gemäß einer weiteren Ausführungsform des vorliegenden Ansatzes kann ein Fluidbehälter als weitere Bodenausnehmung in dem Bodenelement ausgebildet sein. Hierbei kann zumindest ein weiterer Druckkanal in dem Deckelelement ausgebildet sein, um einen Druck in den Fluidbehälter zu leiten. Ferner kann in dem Deckelelement zumindest ein Verbindungskanal ausgebildet sein, um den Fluidbehälter und den Druckkanal fluidisch zu koppeln. Der Fluidbehälter kann ausgebildet sein, um den Druck über den Verbindungskanal und den Druckkanal in den Bereich der Fluidaufnahmefläche zu leiten. Unter einem Fluidbehälter kann beispielsweise eine mit einer Flüssigkeit gefüllte Vertiefung in dem Bodenelement verstanden werden. Bei der Flüssigkeit kann es sich insbesondere um Wasser handeln. Indem das Wasser über der ausgelenkten Folie als eine Art Diffusionsbarriere fungiert, kann eine Verdunstung einer in den Mikrokavitäten befindlichen Flüssigkeit verringert werden. Ferner bietet diese Ausführungsform den Vorteil einer vereinfachten Handhabung der Analyseeinheit, da keine Flüssigkeit von außen zugeführt werden muss.

Gemäß einer weiteren Ausführungsform kann die Folie auch im Bereich des Fluidbehälters angeordnet sein, um den Fluidbehälter fluiddicht zu verschließen. Hierbei kann der Druck durch eine Auslenkung der Folie auf die Flüssigkeit wirken.

Die Analyseeinheit kann gemäß einer weiteren Ausführungsform des vorliegenden Ansatzes ferner ein Mittel aufweisen, das ausgebildet ist, um einen Druck in den Druckkanal und/oder den weiteren Druckkanal einzuleiten. Unter einem Mittel kann etwa eine Pumpe verstanden werden, die ausgebildet ist, um ein Fluid in den Druckkanal und/oder den weiteren Druckkanal zu pumpen. Bei dem Fluid kann es sich insbesondere um eine Flüssigkeit handeln. Durch das Mittel kann ein zum Auslenken der Folie erforderlicher Druck zuverlässig bereitgestellt werden. Eine Verwendung der Flüssigkeit bietet den zusätzlichen Vorteil einer Verringerung der Dampfdurchlässigkeit der Folie, sofern die Folie im Bereich der Mikrokavitäten durch die Flüssigkeit bedeckt wird.

Der vorliegende Ansatz schafft ferner ein Verfahren zum Betreiben einer Analyseeinheit gemäß einer der vorangehend beschriebenen Ausführungsformen, wobei das Verfahren folgende Schritte umfasst:
Aufbringen eines Fluids auf die Fluidaufnahmefläche; und
Einleiten eines Drucks in den Druckkanal, um das Fluid von der Fluidaufnahmefläche in die Anordnung aus Mikrokavitäten zu bewegen.

Schließlich schafft der vorliegende Ansatz ein Verfahren zum Herstellen einer Analyseeinheit gemäß einer der vorangehend beschriebenen Ausführungsformen, wobei das Verfahren folgende Schritte umfasst:
Bereitstellen eines Bodenelements mit zumindest einer Bodenausnehmung, die eine Fluidaufnahmefläche und eine Anordnung aus Mikrokavitäten aufweist, eines Deckelelements mit zumindest einem Druckkanal, der ausgebildet ist, um einen Druck in den Bereich der Fluidaufnahmefläche zu leiten, sowie einer Folie, die ausgebildet ist, um bei Leiten des Drucks in den Bereich der Fluidaufnahmefläche durch den Druck derart verformt zu werden, dass ein Fluid von der Fluidaufnahmefläche in die Anordnung aus Mikrokavitäten bewegt wird;
Zusammenfügen des Deckelelements, des Bodenelements und der Folie, wobei das Zusammenfügen derart erfolgt, dass die Bodenausnehmung dem Deckelelement gegenüberliegt und die Folie im Bereich der Bodenausnehmung zwischen dem Deckelelement und dem Bodenelement angeordnet ist; und
Bilden zumindest eines Fluidkanals zwischen dem Deckelelement und dem Bodenelement, um das Fluid auf die Fluidaufnahmefläche der Bodenausnehmung zu leiten.

Die Erfindung wird nachstehend anhand der beigefügten Zeichnungen beispielhaft näher erläutert. Es zeigen:
- Fig. 1a, 1b, 1c: Querschnittsdarstellungen einer Analyseeinheit gemäß einem Ausführungsbeispiel der vorliegenden Erfindung;
- Fig. 2: eine Aufsichtdarstellung einer Analyseeinheit gemäß einem Ausführungsbeispiel der vorliegenden Erfindung;
- Fig. 3: eine Aufsichtdarstellung einer Analyseeinheit gemäß einem Ausführungsbeispiel der vorliegenden Erfindung;
- Fig. 4a, 4b: Querschnittsdarstellungen eines Bodenelements mit einem Trennelement gemäß verschiedenen Ausführungsbeispielen der vorliegenden Erfindung;
- Fig. 5: eine Querschnittsdarstellung einer Analyseeinheit gemäß einem Ausführungsbeispiel der vorliegenden Erfindung;
- Fig. 6: eine Querschnittsdarstellung einer Analyseeinheit gemäß einem Ausführungsbeispiel der vorliegenden Erfindung;
- Fig. 7a, 7b: Querschnittsdarstellungen einer Analyseeinheit gemäß einem Ausführungsbeispiel der vorliegenden Erfindung;
- Fig. 8: eine Querschnittsdarstellung einer Analyseeinheit gemäß einem Ausführungsbeispiel der vorliegenden Erfindung;
- Fig. 9: eine Querschnittsdarstellung einer Analyseeinheit gemäß einem Ausführungsbeispiel der vorliegenden Erfindung;
- Fig. 10: ein Ablaufdiagramm eines Verfahrens zum Betreiben einer Analyseeinheit gemäß einem Ausführungsbeispiel der vorliegenden Erfindung; und
- Fig. 11: ein Ablaufdiagramm eines Verfahrens zum Herstellen einer Analyseeinheit gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

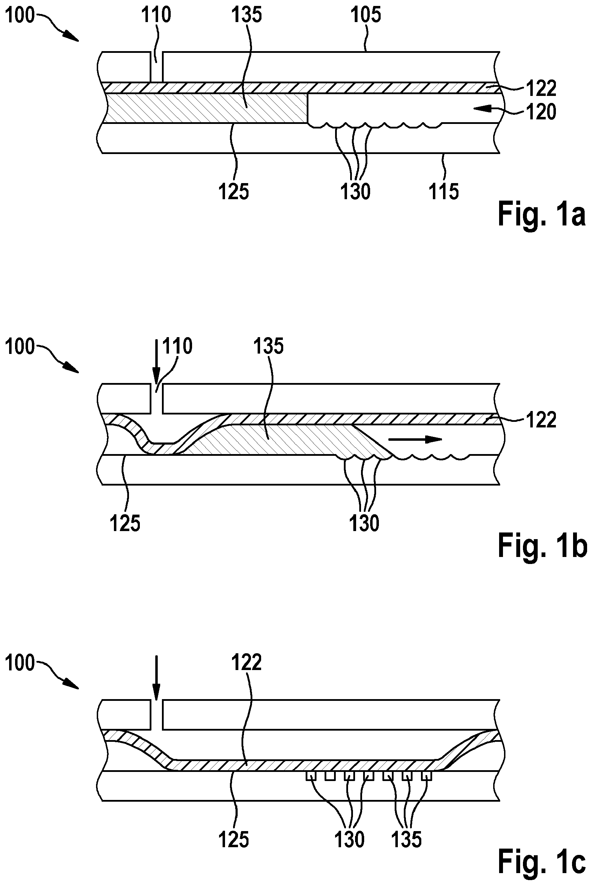

Die Figuren 1a, 1b und 1c zeigen Querschnittsdarstellungen einer Analyseeinheit 100 gemäß einem Ausführungsbeispiel der vorliegenden Erfindung. Die Analyseeinheit 100 umfasst ein Deckelelement 105 mit einem Druckkanal 110, ein Bodenelement 115 mit einer Bodenausnehmung 120 sowie eine Folie 122. Der Druckkanal 110 ist gemäß diesem Ausführungsbeispiel als Durchgangsöffnung in dem Deckelelement 105 ausgebildet. Die Bodenausnehmung 120 weist eine Fluidaufnahmefläche 125 sowie eine Anordnung aus Mikrokavitäten 130 auf. Die Folie 122 ist zwischen dem Deckelelement 105 und dem Bodenelement 150 im Bereich der Bodenausnehmung 120 angeordnet. Die Bodenausnehmung 120 ist derart angeordnet, dass die Fluidaufnahmefläche 125 dem Druckkanal 110 gegenüberliegt und die Mikrokavitäten 130 seitlich zum Druckkanal 110 versetzt sind. Auf der Fluidaufnahmefläche 125 befindet sich ein Fluid 135, dessen Volumen einen Hohlraum zwischen der Fluidaufnahmefläche 125 und der Folie 122 ausfüllt. Das Fluid 135 erstreckt sich ferner bis zu einem an die Anordnung aus Mikrokavitäten 130 angrenzenden Randbereich der Fluidaufnahmefläche 125. Um das Fluid 135 auf die Fluidaufnahmefläche 125 zu leiten, ist zwischen dem Deckelelement 105 und dem Bodenelement 115 ein Fluidkanal (nicht sichtbar) ausgebildet.

Die Folie 122 ist ausgebildet, um bei Anlegen eines Drucks an dem Druckkanal 110 durch den Druck derart verformt zu werden, dass das Fluid 135 von der Fluidaufnahmefläche 125 in die Mikrokavitäten 130 bewegt wird.

Fig. 1a zeigt die Analyseeinheit 100 in einem nicht aktivierten Zustand.

Fig. 1b zeigt die Analyseeinheit 100 bei Anlegen des Drucks an dem Druckkanal 110. Eine Richtung des Drucks ist mit einem Pfeil gekennzeichnet. Die Folie 122 wird durch den Druck derart ausgewölbt, dass sie auf der Fluidaufnahmefläche 125 aufliegt. Zunächst liegt die Folie 122 lediglich im Bereich der Durchgangsöffnung 110 auf der Fluidaufnahmefläche 125 auf. Dies hat zur Folge, dass das Fluid 135 von der Fluidaufnahmefläche 125 in Richtung der Mikrokavitäten 130 verdrängt wird, sodass einige der Mikrokavitäten 130 mit dem Fluid 135 befüllt werden. Eine Bewegungsrichtung des Fluids 135 ist mit einem weiteren Pfeil dargestellt.

Durch den Druck wird die Folie 122 kontinuierlich in Richtung der Mikrokavitäten 130 ausgewölbt, bis sie ganzflächig auf der Fluidaufnahmefläche 125 und den Mikrokavitäten 130 aufliegt, wie in Fig. 1c gezeigt.

Fig. 1c zeigt die Analyseeinheit 100 in einem vollständig aktivierten Zustand. Hierbei sind alle Mikrokavitäten 130 mit dem Fluid 135 befüllt und durch die Folie 122 abgedeckt.

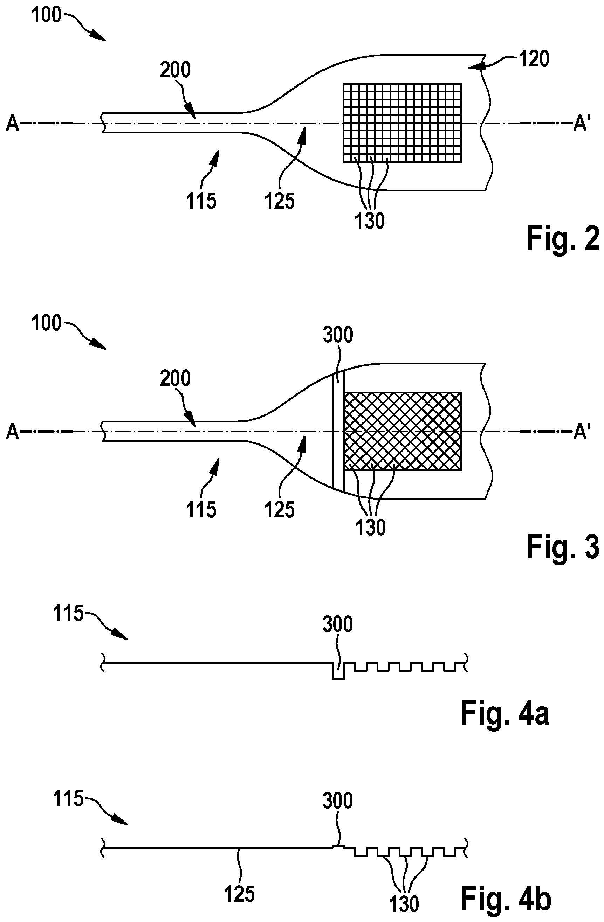

Fig. 2 zeigt eine Aufsichtdarstellung einer Analyseeinheit 100 gemäß einem Ausführungsbeispiel der vorliegenden Erfindung. Das Bodenelement 115 weist einen Fluidkanal 200 auf, der in die Bodenausnehmung 120 mündet und ausgebildet ist, um das Fluid auf die Fluidaufnahmefläche 125 zu leiten. Eine Schnittachse der in den Figuren 1a bis 1c gezeigten Querschnittsdarstellungen ist durch eine Linie AA' gekennzeichnet.

Gemäß einem Ausführungsbeispiel der vorliegenden Erfindung besteht ein Schichtaufbau der Analyseeinheit 100 aus einem ersten Polymersubstrat 105 mit einem Durchloch 110, an dessen Unterseite eine auslenkbare Polymermembran 122 angebracht ist. Planparallel zu diesem Aufbau ist ein zweites Polymersubstrat 115 angeordnet, das ein Array aus Mikrokavitäten 130 trägt. Durch diese Anordnung wird ein mikrofluidischer Kanal als Fluidkanal 200 realisiert, der sich zu einer Flachkammer 120 aufweitet, in der sich das Kavitätenarray, auch Anordnung aus Mikrokavitäten 130 genannt, befindet.

Das Polymersubstrat 105 kann auch als Deckelelement 105, das Durchloch 110 auch als Druckkanal 110 oder Durchgangsöffnung, das zweite Polymersubstrat 115 auch als Bodenelement 115, die auslenkbare Polymermembran 122 auch als Folie 122 oder Membran 122 und die Flachkammer 120 auch als Bodenausnehmung 120 bezeichnet werden.

Um den Auslenkvorgang der Polymermembran 122 kontrolliert von links nach rechts ablaufen zu lassen, ist es besonders vorteilhaft, zwischen der Polymermembran 122 und dem Polymersubstrat 105 eine Haftverbindung herzustellen, die derart ausgeprägt ist, dass sich die Verbindung durch die Druckbeaufschlagung zunächst im Bereich des Durchlochs 110 und anschließend langsam von links nach rechts löst. Eine solche einmalig reversible Verbindung kann beispielsweise mittels Laserdurchstrahlschweißen erfolgen.

Gemäß einem weiteren Ausführungsbeispiel ist ein Array aus Kavitäten 130 in einen polymeren Schichtaufbau integriert. Die auslenkbare Membran 122 des Aufbaus wird für die Befüllung des Kavitätenarrays mit einer zu untersuchenden Probenlösung 135 und gleichzeitig zum Abdecken des Arrays und Abdichten der Arraykavitäten 130 verwendet. Die Probenlösung 135 kann auch als Fluid 135, Probenvolumen 135 oder Probe 135 bezeichnet werden. Dabei befindet sich das Kavitätenarray in der gleichen Ebene wie die mikrofluidischen Kanäle 200. Die Membran 122 befindet sich darüber.

Die Probenlösung 135 wird durch einen Kanal an eine Kante des Arrays geleitet und dort gestoppt. Durch Auslenkung der elastischen Membran 122 wird der Meniskus der Probenlösung 135 kontrolliert über das Kavitätenarray geschoben, wobei sich die Kavitäten 130 kapillar befüllen, wie beispielhaft in den Figuren 1 b und 1c gezeigt.

Während der Polymerasekettenreaktion bleibt die Membran 122 ausgelenkt bzw. abgesenkt, wodurch ein Array aus Kavitäten 130 für eigenständige Polymerasekettenreaktionen aufrechterhalten wird.

Ein weiteres Ausführungsbeispiel der vorliegenden Erfindung sieht eine Einbindung des Kavitätenarrays in Lab-on-a-chip-Systeme für den Nachweis von Nukleinsäuren mittels digitaler Polymerasekettenreaktion vor. Ein solches System in Form eines mikrofluidischen Chips mit integriertem Kavitätenarray erlaubt die Durchführung einer Probenvorbereitung zusammen mit einer nachgeschalteten digitalen Polymerasekettenreaktion auf einem polymeren Lab-on-a-chip-Mehrschichtsystem.

### Hierdurch ergeben sich folgende Vorteile:

Zusammen mit einer in dem gleichen Lab-on-a-chip-System durchgeführten Probenvorbereitung ergibt sich ein vollintegriertes diagnostisches System, das DNA-Moleküle in kleinsten Konzentrationen nachweisen kann.

Die flexible Polymermembran 122 des Mehrschichtaufbaus erlaubt eine kontrollierte und automatisierte Befüllung der Kavitäten 130. Hierdurch werden Bedienungsfehler ausgeschlossen und es kann auf speziell trainiertes Personal zur Befüllung der Kavitäten 130 verzichtet werden.

Durch die Sensitivität der digitalen Polymerasekettenreaktion erweitert sich der Einsatzbereich von Lab-on-a-chip-Systemen zum Beispiel auf die nichtinvasive pränatale Diagnostik, den Nachweis zirkulierender Tumorzellen bzw. zellfreier Tumor-DNA, den Nachweis von Punktmutationen für die personalisierte Medizin sowie die Detektion kleinster Veränderungen in der Genexpression.

Die Kombination aus Probenvorbereitung und digitaler Polymerasekettenreaktion spart manuelle Handhabungsschritte und Zeit.

Es kann eine Kontaminationsgefahr durch verschleppte Polymerasekettenreaktionsprodukte verringert werden, da keine aufgereinigten Produkte manuell transferiert werden müssen.

Gemäß Stand der Technik können DNA-Moleküle in einem zweischrittigen Verfahren aus Endpunkt-Polymerasekettenreaktion und anschließender Mikroarray-Analyse nachgewiesen werden. Im Vergleich dazu bietet das vorgeschlagene Verfahren einen schnelleren Prozessablauf und eine vereinfachte Prozessführung.

Fig. 3 zeigt eine Aufsichtdarstellung einer Analyseeinheit 100 gemäß einem Ausführungsbeispiel der vorliegenden Erfindung. Im Gegensatz zu Fig. 2 weist die in Fig. 3 dargestellte Analyseeinheit 100 ein optionales Trennelement 300 auf. Das Trennelement 300 ist quer zur Linie AA' angeordnet. Gemäß diesem Ausführungsbeispiel ist das Trennelement 300 als Rille in dem Bodenelement 115 ausgebildet, wobei sich die Rille über eine gesamte Breite der Bodenausnehmung 120 erstreckt. Ferner ist das Trennelement 300 entlang einer der Fluidaufnahmefläche 125 benachbarten Kante der Anordnung aus Mikrokavitäten 130 angeordnet.

Das Trennelement 300 ist ausgebildet, um ein Bewegen des Fluids von der Fluidaufnahmefläche 125 in die Mikrokavitäten 130 vor Anlegen des Drucks an der Durchgangsöffnung zu verhindern.

Die Figuren 4a und 4b zeigen Querschnittsdarstellungen eines Bodenelements 115 mit einem Trennelement 300 gemäß verschiedenen Ausführungsbeispielen der vorliegenden Erfindung.

Fig. 4a zeigt ein Ausführungsbeispiel, bei dem das Trennelement 300, wie in Fig. 3 gezeigt, als Rille oder Graben ausgebildet ist.

Fig. 4b zeigt ein Ausführungsbeispiel, bei dem das Trennelement 300 im Gegensatz zu den Figuren 3 und 4a als hydrophober Streifen in einem den Mikrokavitäten 130 benachbarten Bereich der Fluidaufnahmefläche 125 aufgebracht ist.

Gemäß verschiedenen Ausführungsbeispielen der vorliegenden Erfindung weist eine linke Kante des Kavitätenarrays ein Merkmal als Trennelement 300 auf, das einen definierten Stopp des Probenvolumens bedingt. Das Merkmal kann zum einen als hydrophober Stopp, wie in Fig. 4b gezeigt, zum anderen als geometrischer Stopp, etwa als ein in den Figuren 3 und 4a gezeigter Graben, realisiert sein. Durch diese Ausformung lässt sich ein definierter Startpunkt für die Verteilung des Probenvolumens auf die Kavitäten 130 erreichen.

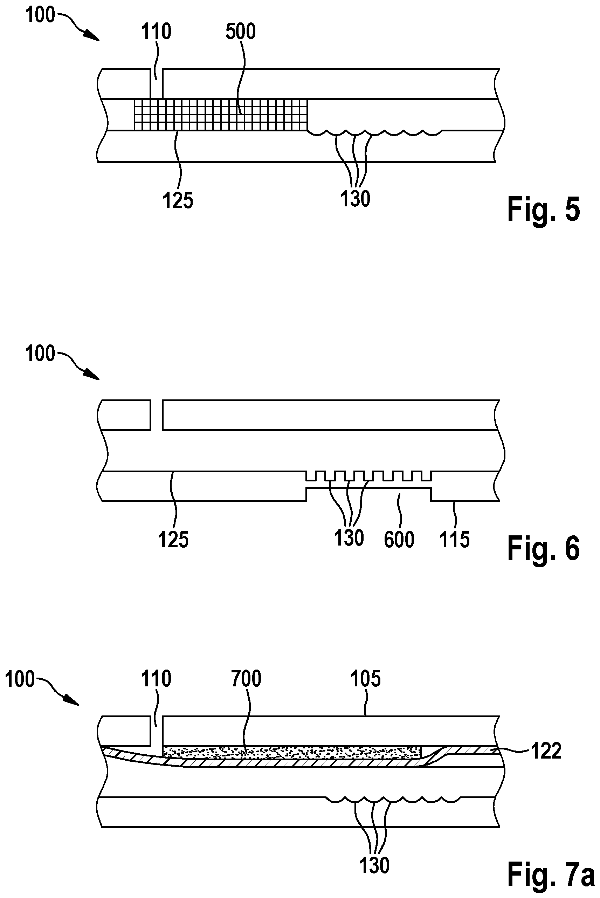

Fig. 5 zeigt eine Querschnittsdarstellung einer Analyseeinheit 100 gemäß einem Ausführungsbeispiel der vorliegenden Erfindung. Im Gegensatz zu den vorangehend beschriebenen Figuren 1a bis 4b ist die in Fig. 5 gezeigte Fluidaufnahmefläche 125 vom Bereich des Druckkanals 110 bis zum Randbereich der Fluidaufnahmefläche 125 mit einer komprimierbaren Faserschicht 500 bedeckt. Die Faserschicht 500 ist ausgebildet, um das Fluid aufzusaugen und bei Anlegen des Drucks am Druckkanal 110 durch die Folie derart komprimiert zu werden, dass das Fluid in Richtung der Mikrokavitäten 130 wieder abgegeben wird.

In einer weiteren Ausführungsform enthalten Teile des Kanals und der Flachkammer ein saugfähiges Vlies als Faserschicht 500, auch Schwamm genannt. Dies hat den Vorteil, dass der Schwamm ein definiertes Volumen aufnehmen kann. Durch Aktuierung der Polymermembran kann das in dem Vlies befindliche Volumen in das Kavitätenarray eingefüllt werden. Dadurch wird nicht nur ein Startpunkt für die Befüllung definiert, sondern auch gleichzeitig ein Probenvolumen abgemessen.

Fig. 6 zeigt eine Querschnittsdarstellung einer Analyseeinheit 100 gemäß einem Ausführungsbeispiel der vorliegenden Erfindung. Im Gegensatz zu den vorangehend beschriebenen Figuren weist das in Fig. 6 gezeigte Bodenelement 115 im Bereich der Mikrokavitäten 130 eine Wandstärke auf, die geringer ist als eine übrige Wandstärke des Bodenelements 115. Dadurch wird ein leichterer Zugang zu den Mikrokavitäten 130 ermöglicht, etwa um das in den Mikrokavitäten 130 enthaltene Fluid zu temperieren und/oder zu analysieren.

Optional ist eine Abdünnung als Aussparung 600 des Polymersubstrats 115 unterhalb des Kavitätenarrays ausgebildet. Erstens kann dadurch thermische Energie für die Durchführung der temperaturgesteuerten Polymerasekettenreaktion schnell in den Bereich des Kavitätenarrays eingebracht werden. Zweitens kann dadurch eine Ausleseoptik in einer geringen physikalischen Distanz zum Kavitätenarray angebracht werden, wodurch sich der Absorptionsanteil des Polymersubstrats 115 verringert.

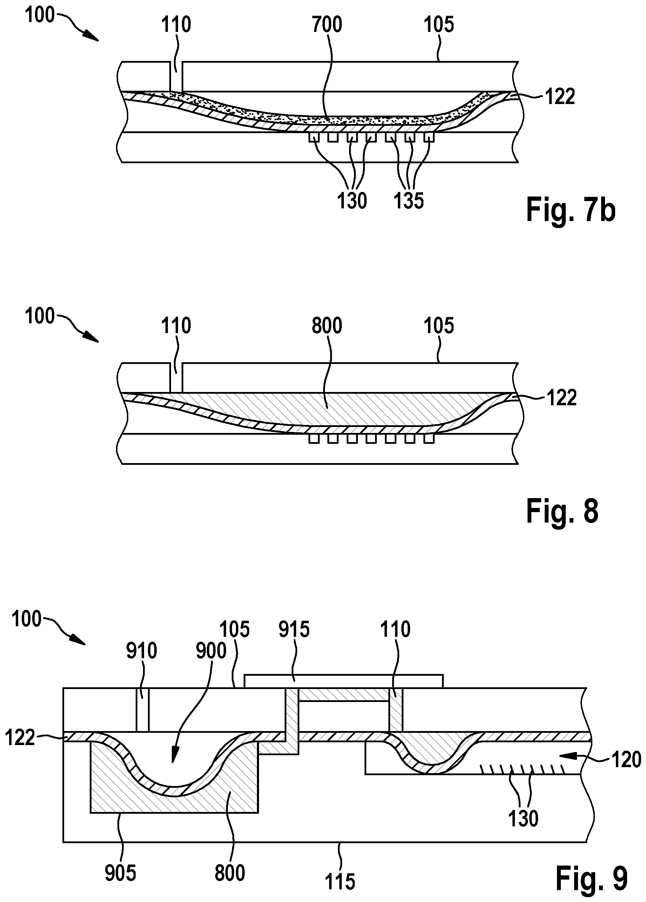

Die Figuren 7a und 7b zeigen Querschnittsdarstellungen einer Analyseeinheit 100 gemäß einem Ausführungsbeispiel der vorliegenden Erfindung. Die in den Figuren 7a und 7b dargestellte Folie 122 weist im Gegensatz zu den vorangehend beschriebenen Figuren eine zusätzliche Isolierschicht 700 auf. Gemäß diesem Ausführungsbeispiel ist die Isolierschicht 700 zwischen der Folie 122 und dem Deckelelement 105 ausgebildet. Hierbei erstreckt sich die Isolierschicht 700 vom Bereich der Durchgangsöffnung 110 bis zu einem der Durchgangsöffnung 110 abgewandten Randbereich der Anordnung aus Mikrokavitäten 130. Die Isolierschicht 700 ist ausgebildet, um eine Dampfdurchlässigkeit der Folie 122 zu verringern.

Fig. 7a zeigt die Folie 122 in einem nicht ausgelenkten Zustand. Fig. 7b zeigt die Folie 122 in einem ausgelenkten Zustand, wie bereits anhand der Figuren 1a bis 1c beschrieben.

Zwischen dem Polymersubstrat 105 und der Polymermembran 122 ist zusätzlich oder alternativ eine dünne Paraffinschicht als Isolierschicht 700 ausgebildet. Nach Auslenkung der Membran 122 und dem Schmelzen der Paraffinschicht wird die ausgelenkte Membran 122 mit dem Paraffin überschichtet, wie in Fig. 7b gezeigt. Die so realisierte Dampfsperre hat den Vorteil, dass sie ohne Verwendung flüssiger Medien auskommt und automatisch aktiviert wird, wenn das System im Betrieb unter Temperatur gesetzt wird. Dies hat außerdem den Vorteil, dass während der Herstellung die Paraffinschicht als Festkörper eingebracht werden kann, womit das Dispensen flüssiger Stoffe entfällt.

Gemäß einem weiteren Ausführungsbeispiel ist die Oberfläche der Polymermembran 122 so beschichtet, dass die Dampfdurchlässigkeit verringert wird. Hier können alle aus dem Stand der Technik bekannten Techniken angewendet werden. Dies sind zum Beispiel Prozesse der chemischen Gasphasenabscheidung (chemical vapour deposition; CVD) oder der physikalischen Gasphasenabscheidung (physical vapour deposition; PVD).

Zusätzlich kann die Polymermembran 122 als Mehrschichtverbund realisiert sein. Durch eine Wahl und Komposition geeigneter Materialien kann damit die Dampfdurchlässigkeit verringert werden. Diese Ausführungsform hat insbesondere den Vorteil, dass der Inhalt der Kavitäten 130 noch besser abgedichtet und damit die Zuverlässigkeit des Systems erhöht wird.

Fig. 8 zeigt eine Querschnittsdarstellung einer Analyseeinheit 100 gemäß einem Ausführungsbeispiel der vorliegenden Erfindung. Die Folie 122 ist in Fig. 8 in einem ausgelenkten Zustand gezeigt. Hierbei sind die Mikrokavitäten 130 durch die Folie 122 fluiddicht verschlossen. Gemäß diesem Ausführungsbeispiel ist die Analyseeinheit 100 mit einem hier nicht dargestellten Mittel zum Einleiten eines Drucks in den Druckkanal 110 ausgestattet. Das Mittel ist ausgebildet, um eine Flüssigkeit 800 durch den Druckkanal 110 zu pumpen, sodass die Folie 122 durch den Druck der Flüssigkeit 800 ausgewölbt wird. Ein durch das Auswölben der Folie 122 entstehender Hohlraum zwischen der Folie 122 und dem Deckelelement 105 wird hierbei durch die Flüssigkeit 800 gefüllt.

Durch eine Überschichtung der ausgelenkten Polymermembran 122 mit einem von extern zugeführten flüssigen Medium 800, auch Flüssigkeit 800 genannt, lässt sich die Verdunstung des in den Kavitäten 130 befindlichen Probenvolumens 135 zusätzlich verringern. In dieser Ausführungsform kann auf eine Beschichtung oder einen speziellen Schichtaufbau der Polymermembran 122 verzichtet werden.

Das flüssige Medium 800 kann beispielsweise Wasser sein. Bei entsprechender Beschaffenheit der Membran 122 ist in diesem Fall ein Flüssigkeitsaustausch von Wasser durch die Membran 122 hindurch möglich. Die Kavitäten 130 fallen während einer Polymerasekettenreaktion nicht trocken, da ein Flüssigkeitsverlust durch Diffusion von Wasser durch die Membran 122 hindurch unterbunden ist.

Das flüssige Medium 800 kann auch Öl, Fotolack oder ein durch UV oder thermisch aktivierbarer Kleber sein. Durch Überschichten der ausgelenkten Polymermembran 122 mit dem Medium 800 wird eine flüssige Diffusionssperre realisiert und somit Flüssigkeitsverlust unterbunden.

Fig. 9 eine Querschnittsdarstellung einer Analyseeinheit 100 gemäß einem Ausführungsbeispiel der vorliegenden Erfindung. Hierbei umfasst die Analyseeinheit 100 einen Fluidbehälter 900 als weitere Bodenausnehmung 905 in dem Bodenelement 115. Zwischen der weiteren Bodenausnehmung 905 und dem Deckelelement 105 ist die Folie 122 angeordnet. Der Fluidbehälter 900 ist mit einer Flüssigkeit gefüllt und durch die Folie 122 fluiddicht verschlossen. Das Deckelelement weist im Bereich des Fluidbehälters 900 einen weiteren Druckkanal 910 auf. Gemäß diesem Ausführungsbeispiel ist der weitere Druckkanal 910 als eine weitere Durchgangsöffnung in dem Deckelement 105 realisiert. Ferner ist in dem Deckelelement 105 ein Verbindungskanal 915 ausgebildet, um den Fluidbehälter 900 und den Druckkanal 110 fluidisch zu koppeln.

Wie in Fig. 9 gezeigt, kann durch den weiteren Druckkanal 910 etwa mittels einer Pumpe (nicht dargestellt) ein Druck auf die Folie 122 ausgeübt werden, sodass die Folie 122 im Bereich des Fluidbehälters 900 ausgelenkt wird. Mittels der Flüssigkeit wird der Druck über den Verbindungskanal 915 und den Druckkanal 110 auf einen im Bereich der Bodenausnehmung 120 befindlichen Teil der Folie 122 gelenkt, sodass die Folie 122 auch in diesem Bereich ausgelenkt wird. Hierbei wird das Fluid in die Mikrokavitäten 130 gedrückt.

Ein Ausführungsbeispiel der vorliegenden Erfindung sieht eine Integration eines Reservoirs als Fluidbehälter 900 für das anhand von Fig. 8 beschriebene flüssige Medium 800 vor. Durch die Beaufschlagung dieses Reservoirs mit Druck wird das Medium 800 in den Zuführungskanal 915, auch Verbindungskanal 915 genannt, geleitet. Das Medium 800 lenkt dann die Polymermembran 122 aus, wodurch wiederum das Kavitätenarray dampfdicht verschlossen wird.

Diese Ausführung hat den Vorteil, dass das Medium nicht von außen zugeführt werden muss, was die Prozessführung vereinfacht. Da das Medium 800 in dieser Ausführungsform ohne direkten Zugang zur Außenwelt ist, ergibt sich außerdem der weitere Vorteil, dass das Medium 800 auch nach Prozessende bei Wegnahme aller Aktuierungsdrücke nicht austreten und beispielsweise eine externe Steuereinheit verschmutzen kann.

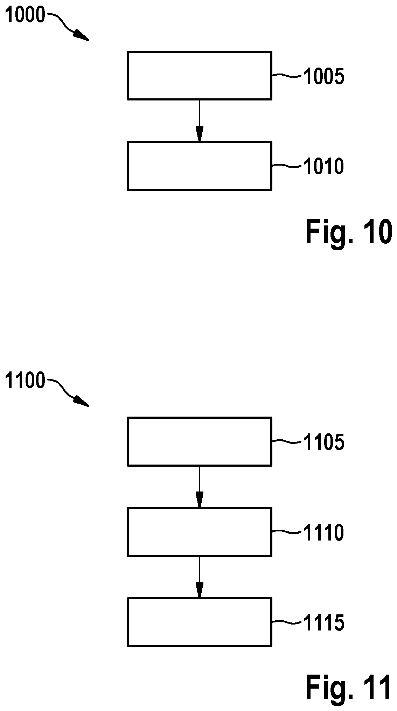

Fig. 10 zeigt ein Ablaufdiagramm eines Verfahrens 1000 zum Betreiben einer Analyseeinheit gemäß einem Ausführungsbeispiel der vorliegenden Erfindung. In einem Schritt 1005 wird zunächst ein Fluid auf die Fluidaufnahmefläche aufgebracht. Anschließend erfolgt in einem Schritt 1010 das Einleiten eines Drucks in den Druckkanal, um das Fluid von der Fluidaufnahmefläche in die Anordnung aus Mikrokavitäten zu bewegen.

Gemäß einem Ausführungsbeispiel der vorliegenden Erfindung wird das zu untersuchende Probenvolumen 135 in den Kanal 200 und einen Teil der Flachkammer 120 geleitet und dort gestoppt. An das Durchloch 110 wird ein Überdruck angelegt, wodurch die Polymermembran 122 ausgelenkt wird. Dabei senkt sich die Polymermembran 122 von links nach rechts ab. Dadurch wird das darunter befindliche Probenvolumen 135 in Richtung des Kavitätenarrays 130 bewegt, wie in Fig. 1b dargestellt. Sobald die Membran 122 komplett abgesenkt ist, haben sich alle Kavitäten 130 kapillar mit der Probe 135 befüllt (siehe Figuren 1c, 7b und 8). Während der Durchführung der Polymerasekettenreaktion bleibt die Membran 122 abgesenkt, wodurch die Kavitäten 130 verschlossen bleiben.

Fig. 11 zeigt ein Ablaufdiagramm eines Verfahrens 1100 zum Herstellen einer Analyseeinheit gemäß einem Ausführungsbeispiel der vorliegenden Erfindung. In einem Schritt 1105 werden ein Bodenelement mit zumindest einer Bodenausnehmung, die eine Fluidaufnahmefläche und eine Anordnung aus Mikrokavitäten aufweist, ein Deckelelement mit zumindest einem Druckkanal, der ausgebildet ist, um einen Druck in den Bereich der Fluidaufnahmefläche zu leiten, sowie eine Folie bereitgestellt. Hierbei ist die Folie ausgebildet, um bei Leiten des Drucks in den Bereich der Fluidaufnahmefläche durch den Druck derart verformt zu werden, dass ein Fluid von der Fluidaufnahmefläche in die Anordnung aus Mikrokavitäten bewegt wird.

In einem weiteren Schritt 1110 erfolgt das Zusammenfügen des Deckelelements, des Bodenelements und der Folie. Hierbei erfolgt das Zusammenfügen derart, dass die Bodenausnehmung dem Deckelelement gegenüberliegt und die Folie im Bereich der Bodenausnehmung zwischen dem Deckelelement und dem Bodenelement angeordnet ist.

Schließlich wird in einem Schritt 1115 zumindest ein Fluidkanal zwischen dem Deckelelement und dem Bodenelement gebildet, um das Fluid auf die Fluidaufnahmefläche der Bodenausnehmung zu leiten.

Die nötigen Strukturen in den Polymersubstraten 105, 115 können beispielsweise durch Fräsen, Spritzguss, Heißprägen oder Laserstrukturierung erzeugt werden. Das Kavitätenarray kann entweder direkt im Polymer ausgeformt sein oder als Einlegeteil, beispielsweise aus Glas hergestellt, in den Polymerschichtaufbau eingebracht werden.

Als Materialbeispiele können für das Polymersubstrat Thermoplaste wie beispielsweise Polycarbonat (PC), Polypropylen (PP), Polyethylen (PE), Polymethylmethacrylat (PMMA), Cyclo-Polefin-Polymer (COP) oder Cyclo-Polefin-Copolymer (COC) eingesetzt werden und für die Polymermembran Elastomer, thermoplastisches Elastomer auf Urethanbasis (TPU), Styrol-Blockcopolymere (TPS), Thermoplaste, Heißklebefolien oder Siegelfolien für Mikrotiterplatten.

Als beispielhafte Abmessungen der Ausführungsbeispiele können die Dicke des Polymersubstrats 0,5 bis 5 mm, der Kanaldurchmesser in den Polymersubstraten 10 µm bis 3 mm, die Dicke der Polymermembran 5 bis 500 µm und die laterale Abmessungen des gesamten Ausführungsbeispiels 10 × 10 mm² bis 200 × 200 mm² betragen.

Das Volumen der Kavitäten des Arrays kann 1 fL bis 100 µL sein.

Die Anzahl der Kavitäten kann beispielsweise von 10¹ bis 10⁹ reichen.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können erfindungsgemäße Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Analyseeinheit (100) zum Durchführen einer Polymerasekettenreaktion, wobei die Analyseeinheit (100) folgende Merkmale aufweist:
ein Deckelelement (105);
ein Bodenelement (115) mit zumindest einer Bodenausnehmung (120), wobei die Bodenausnehmung (120) eine Fluidaufnahmefläche (125) und eine Anordnung aus Mikrokavitäten (130) aufweist, wobei die Bodenausnehmung (120) dem Deckelelement (105) gegenüberliegend angeordnet ist;
zumindest einen Fluidkanal (200), der zwischen dem Deckelelement (105) und dem Bodenelement (115) ausgebildet ist, um ein Fluid (135) auf die Fluidaufnahmefläche (125) der Bodenausnehmung (120) zu leiten;
zumindest einen Druckkanal (110), der in dem Deckelelement (105) ausgebildet ist, um einen Druck in einen Bereich der Fluidaufnahmefläche (125) zu leiten; und
eine Folie (122), die im Bereich der Bodenausnehmung (120) zwischen dem Deckelelement (105) und dem Bodenelement (115) angeordnet ist, wobei die Folie (122) ausgebildet ist, um bei Leiten des Drucks in den Bereich der Fluidaufnahmefläche (125) durch den Druck derart verformt zu werden, dass das Fluid (135) von der Fluidaufnahmefläche (125) in die Anordnung aus Mikrokavitäten (130) bewegt wird.

2. Analyseeinheit (100) gemäß Anspruch 1, **dadurch gekennzeichnet, dass** der Druckkanal (110) als Durchgangsöffnung in dem Deckelelement (105) ausgebildet ist, wobei die Bodenausnehmung (120) derart angeordnet ist, dass die Fluidaufnahmefläche (125) der Durchgangsöffnung gegenüberliegt und die Anordnung aus Mikrokavitäten (130) seitlich zur Durchgangsöffnung versetzt ist.

3. Analyseeinheit (100) gemäß einem der vorangegangenen Ansprüche, **dadurch gekennzeichnet, dass** die Folie (122) zumindest im Bereich der Fluidaufnahmefläche (125) und/oder im Bereich der Anordnung aus Mikrokavitäten (130) lösbar an dem Deckelelement (105) befestigt ist.

4. Analyseeinheit (100) gemäß einem der vorangegangenen Ansprüche, **dadurch gekennzeichnet, dass** die Folie (122) ferner ausgebildet ist, um durch den Druck derart verformt zu werden, dass die Anordnung aus Mikrokavitäten (130) fluiddicht verschlossen wird.

5. Analyseeinheit (100) gemäß einem der vorangegangenen Ansprüche, **dadurch gekennzeichnet, dass** zwischen der Anordnung aus Mikrokavitäten (130) und der Fluidaufnahmefläche (125) ein Trennelement (300) ausgebildet ist, um ein Bewegen des Fluids (135) von der Fluidaufnahmefläche (125) in die Anordnung aus Mikrokavitäten (130) vor Leiten des Drucks in den Bereich der Fluidaufnahmefläche (125) zu verhindern.

6. Analyseeinheit (100) gemäß einem der vorangegangenen Ansprüche, **dadurch gekennzeichnet, dass** die Fluidaufnahmefläche (125) zumindest teilweise mit einer komprimierbaren Faserschicht (500) zum Aufnehmen und/oder Abgeben des Fluids (135) bedeckt ist.

7. Analyseeinheit (100) gemäß einem der vorangegangenen Ansprüche, **dadurch gekennzeichnet, dass** das Bodenelement (115) im Bereich der Anordnung aus Mikrokavitäten (130) eine vorbestimmte Maximaldicke aufweist.

8. Analyseeinheit (100) gemäß einem der vorangegangenen Ansprüche, **dadurch gekennzeichnet, dass** die Folie (122) zumindest im Bereich der Anordnung aus Mikrokavitäten (130) eine Isolierschicht (700) aufweist und/oder als Mehrschichtverbund ausgebildet ist, um eine Dampfdurchlässigkeit der Folie (122) zu verringern.

9. Analyseeinheit (100) gemäß Anspruch 8, **dadurch gekennzeichnet, dass** die Isolierschicht (700) auf einer dem Deckelelement (105) zugewandten Seite der Folie (122) ausgebildet ist, insbesondere wobei die Isolierschicht (700) aus Paraffin gebildet ist.

10. Analyseeinheit (100) gemäß einem der vorangegangenen Ansprüche, **dadurch gekennzeichnet, dass** ein Fluidbehälter (900) als weitere Bodenausnehmung (905) in dem Bodenelement (115) ausgebildet ist, wobei zumindest ein weiterer Druckkanal (910) in dem Deckelelement (105) ausgebildet ist, um einen Druck in den Fluidbehälter (900) zu leiten, wobei zumindest ein Verbindungskanal (915) in dem Deckelelement (105) ausgebildet ist, um den Fluidbehälter (900) und den Druckkanal (110) fluidisch zu koppeln, wobei der Fluidbehälter (900) ausgebildet ist, um den Druck über den Verbindungskanal (915) und den Druckkanal (110) in den Bereich der Fluidaufnahmefläche (125) zu leiten.

11. Analyseeinheit (100) gemäß Anspruch 10, **gekennzeichnet durch** ein Mittel, das ausgebildet ist, um einen Druck in den Druckkanal (110) und/oder den weiteren Druckkanal (910) einzuleiten.

12. Verfahren (1000) zum Betreiben einer Analyseeinheit (100) gemäß einem der Ansprüche 1 bis 11, wobei das Verfahren (1000) folgende Schritte umfasst:
Aufbringen (1005) eines Fluids (135) auf die Fluidaufnahmefläche (125); und
Einleiten (1010) eines Drucks in den Druckkanal (110), um das Fluid (135) von der Fluidaufnahmefläche (125) in die Anordnung aus Mikrokavitäten (130) zu bewegen.

13. Verfahren (1100) zum Herstellen einer Analyseeinheit (100) gemäß einem der Ansprüche 1 bis 11, wobei das Verfahren (1100) folgende Schritte umfasst:
Bereitstellen (1105) eines Bodenelements (115) mit zumindest einer Bodenausnehmung (120), die eine Fluidaufnahmefläche (125) und eine Anordnung aus Mikrokavitäten (130) aufweist, eines Deckelelements (105) mit zumindest einem Druckkanal (110), der ausgebildet ist, um einen Druck in den Bereich der Fluidaufnahmefläche (125) zu leiten, sowie einer Folie (122), die ausgebildet ist, um bei Leiten des Drucks in den Bereich der Fluidaufnahmefläche (125) durch den Druck derart verformt zu werden, dass ein Fluid (135) von der Fluidaufnahmefläche (125) in die Anordnung aus Mikrokavitäten (130) bewegt wird;
Zusammenfügen (1110) des Deckelelements (105), des Bodenelements (115) und der Folie (122), wobei das Zusammenfügen (1110) derart erfolgt, dass die Bodenausnehmung (120) dem Deckelelement (105) gegenüberliegt und die Folie (122) im Bereich der Bodenausnehmung (120) zwischen dem Deckelelement (105) und dem Bodenelement (115) angeordnet ist; und
Bilden (1115) zumindest eines Fluidkanals (200) zwischen dem Deckelelement (105) und dem Bodenelement (115), um das Fluid (135) auf die Fluidaufnahmefläche (125) der Bodenausnehmung (120) zu leiten.

## Claims

1. Analysis unit (100) for performing a polymerase chain reaction, wherein the analysis unit (100) has the following features:
a cover element (105);
a bottom element (115) with at least one bottom hollow (120), wherein the bottom hollow (120) has a fluid-receiving surface (125) and an arrangement of microcavities (130), wherein the bottom hollow (120) is arranged lying opposite the cover element (105);
at least one fluid channel (200), which is formed between the cover element (105) and the bottom element (115) in order to convey a fluid (135) onto the fluid-receiving surface (125) of the bottom hollow (120);
at least one pressure channel (110), which is formed in the cover element (105) in order to convey a pressure into an area of the fluid-receiving surface (125); and
a film (122), which is arranged between the cover element (105) and the bottom element (115) in the area of the bottom hollow (120), wherein the film (122) is designed to be deformed by pressure, when the pressure is conveyed into the area of the fluid-receiving surface (125), in such a way that the fluid (135) is moved from the fluid-receiving surface (125) into the arrangement of microcavities (130).

2. Analysis unit (100) according to Claim 1, **characterized in that** the pressure channel (110) is formed as a through-opening in the cover element (105), wherein the bottom hollow (120) is arranged in such a way that the fluid-receiving surface (125) lies opposite the through-opening, and the arrangement of microcavities (130) is laterally offset with respect to the through-opening.

3. Analysis unit (100) according to one of the preceding claims, **characterized in that** the film (122) is secured releasably on the cover element (105) at least in the area of the fluid-receiving surface (125) and/or in the area of the arrangement of microcavities (130).

4. Analysis unit (100) according to one of the preceding claims, **characterized in that** the film (122) is moreover designed to be deformed by the pressure in such a way that the arrangement of microcavities (130) is closed in a fluid-tight manner.

5. Analysis unit (100) according to one of the preceding claims, **characterized in that** a separation element (300) is formed between the arrangement of microcavities (130) and the fluid-receiving surface (125), in order to prevent a movement of the fluid (135) from the fluid-receiving surface (125) into the arrangement of microcavities (130) before the pressure has been conveyed into the area of the fluid-receiving surface (125).

6. Analysis unit (100) according to one of the preceding claims, **characterized in that** the fluid-receiving surface (125) is at least partially covered with a compressible fiber layer (500) for taking up and/or releasing the fluid (135).

7. Analysis unit (100) according to one of the preceding claims, **characterized in that** the bottom element (115) has a predetermined maximum thickness in the area of the arrangement of microcavities (130).

8. Analysis unit (100) according to one of the preceding claims, **characterized in that** the film (122), at least in the area of the arrangement of microcavities (130), has an insulation layer (700) and/or is designed as a multi-layer composite in order to reduce a vapor permeability of the film (122).

9. Analysis unit (100) according to Claim 8, **characterized in that** the insulation layer (700) is formed on a side of the film (122) facing toward the cover element (105), in particular wherein the insulation layer (700) is formed from paraffin.

10. Analysis unit (100) according to one of the preceding claims, **characterized in that** a fluid container (900) is formed as a further bottom hollow (905) in the bottom element (115), wherein at least one further pressure channel (910) is formed in the cover element (105) in order to convey a pressure into the fluid container (900), wherein at least one connection channel (915) is formed in the cover element (105) in order to couple the fluid container (900) and the pressure channel (110) fluidically, wherein the fluid container (900) is designed to convey the pressure through the connection channel (915) and the pressure channel (110) into the area of the fluid-receiving surface (125).

11. Analysis unit (100) according to Claim 10, **characterized by** a means designed to introduce a pressure into the pressure channel (110) and/or the further pressure channel (910).

12. Method (1000) for operating an analysis unit (100) according to one of Claims 1 to 11, wherein the method (1000) comprises the following steps:
applying (1005) a fluid (135) to the fluid-receiving surface (125); and
introducing (1010) a pressure into the pressure channel (110) in order to move the fluid (135) from the fluid-receiving surface (125) into the arrangement of microcavities (130).

13. Method (1100) for producing an analysis unit (100) according to one of Claims 1 to 11, wherein the method (1100) comprises the following steps:
making available (1105) a bottom element (115) with at least one bottom hollow (120) which has a fluid-receiving surface (125) and an arrangement of microcavities (130), a cover element (105) with at least one pressure channel (110) which is designed to convey a pressure into the area of the fluid-receiving surface (125), and a film (122) which is designed to be deformed by pressure, when the pressure is conveyed into the area of the fluid-receiving surface (125), in such a way that a fluid (135) is moved from the fluid-receiving surface (125) into the arrangement of microcavities (130);
joining together (1110) the cover element (105), the bottom element (115) and the film (122), wherein the joining together (1110) takes place in such a way that the bottom hollow (120) lies opposite the cover element (105) and the film (122) is arranged between the cover element (105) and the bottom element (115) in the area of the bottom hollow (120); and
forming (1115) at least one fluid channel (200) between the cover element (105) and the bottom element (115), in order to convey the fluid (135) onto the fluid-receiving surface (125) of the bottom hollow (120).

## Revendications

1. Unité d'analyse (100) permettant de réaliser une réaction en chaîne de polymérase, l'unité d'analyse (100) présentant les caractéristiques suivantes :
un élément de couvercle (105),
un élément de fond (115) doté d'au moins une découpe de fond (120), la découpe de fond (120) présentant une surface (125) de reprise de fluide et un système de microcavités (130), la découpe de fond (120) étant disposée face à l'élément de couvercle (105),
au moins un canal (200) pour fluide formé entre l'élément de couvercle (105) et l'élément de fond (115) pour guider un fluide (135) sur la surface (125) de réception de fluide de la découpe de fond (120),
au moins un canal (110) sous pression formé dans l'élément de couvercle (105) pour exercer une pression sur une partie de la surface (125) de reprise de fluide et
un film (122) disposé entre l'élément de couvercle (105) et l'élément de fond (115) au niveau de la découpe de fond (120), le film (122) étant configuré pour, lors de l'application de la pression au niveau de la surface (125) de réception de fluide, être déformé sous l'action de la pression de telle sorte que le fluide (135) soit déplacé de la surface (125) de réception de fluide jusque dans le système de microcavités (130).

2. Unité d'analyse (100) selon la revendication 1, **caractérisée en ce que** le canal de pression (110) est configuré comme ouverture de passage ménagée dans l'élément de couvercle (105), la découpe de fond (120) étant disposée de telle sorte que la surface (125) de reprise de fluide soit située face à l'ouverture de passage et que le système de microcavités (130) soit décalé latéralement par rapport à l'ouverture de passage.

3. Unité d'analyse (100) selon l'une des revendications précédentes, **caractérisée en ce que** le film (122) est fixé de manière libérable sur l'élément de couvercle (105) au moins au niveau de la surface (125) de reprise de fluide et/ou au niveau du système de microcavités (130).

4. Unité d'analyse (100) selon l'une des revendications précédentes, **caractérisée en ce que** le film (122) est en outre configuré pour être déformé sous l'action de la pression de telle sorte que le système de microcavités (130) soit fermé de manière étanche aux fluides.

5. Unité d'analyse (100) selon l'une des revendications précédentes, **caractérisée en ce qu'**un élément de séparation (300) est formé entre le système de microcavités (130) et la surface (125) de reprise de fluide pour empêcher un déplacement du fluide (135) depuis la surface (125) de reprise de fluide jusque dans le système de microcavités (130) avant l'application d'une pression au niveau de la surface (125) de reprise de fluide.

6. Unité d'analyse (100) selon l'une des revendications précédentes, **caractérisée en ce que** la surface (125) de reprise de fluide est recouverte au moins en partie par une couche compressible (500) de fibres qui reprend et/ou délivre le fluide (135).

7. Unité d'analyse (100) selon l'une des revendications précédentes, **caractérisée en ce que** l'élément de fond (115) présente au niveau du système de microcavités (130) une épaisseur minimale prédéterminée.

8. Unité d'analyse (100) selon l'une des revendications précédentes, **caractérisée en ce qu'**au moins au niveau du système de microcavités (130), le film (122) présente une couche isolante (700) et/ou est configuré comme composite multicouche qui diminue la perméabilité du film (122) à la vapeur d'eau.

9. Unité d'analyse (100) selon la revendication 8, **caractérisée en ce que** la couche isolante (700) est formée sur le côté du film (122) tourné vers l'élément de couvercle (105) et en particulier dans lequel la couche isolante (700) est réalisée en paraffine.

10. Unité d'analyse (100) selon l'une des revendications précédentes, **caractérisée en ce qu'**un récipient (900) à fluide est configuré comme autre découpe (905) du fond dans l'élément de fond (115), au moins un autre canal sous pression (910) étant formé dans l'élément de couvercle (105) pour appliquer une pression dans le récipient (900) à fluide, au moins un canal de liaison (915) étant formé dans l'élément de couvercle (105) pour raccorder à écoulement le récipient (900) à fluide et le canal sous pression (110), le récipient (900) à fluide étant configuré pour appliquer la pression au niveau de la surface (125) de reprise de fluide par l'intermédiaire du canal de liaison (915) et du canal sous pression (110).

11. Unité d'analyse (100) selon la revendication 10, **caractérisée par** un moyen configuré pour appliquer une pression dans le canal (110) sous pression et/ou dans l'autre canal (910) sous pression.

12. Procédé (1000) de conduite d'une unité d'analyse (100) selon l'une des revendications 1 à 11, le procédé (1000) présentant les étapes suivantes :
application (1005) d'un fluide (135) sur la surface (125) de reprise de fluide et
application (1010) d'une pression dans le canal (110) sous pression pour déplacer le fluide (135) depuis la surface (125) de reprise de fluide jusque dans le système de microcavités (130).

13. Procédé (1100) de fabrication d'un appareil d'analyse (100) selon l'une des revendications 1 à 11, le procédé (1100) comportant les étapes suivantes :
préparer (1105) un élément de fond (115) présentant au moins une découpe de fond (120) qui présente une surface (125) de reprise de fluide et un système de microcavités (130), un élément de couvercle (105) présentant au moins un canal (110) sous pression configuré pour appliquer une pression au niveau de la surface (125) de reprise de fluide, ainsi qu'un film (122) configuré pour, lors de l'application de la pression au niveau de la surface (125) de reprise de fluide, être déformé sous l'action de la pression de telle sorte qu'un fluide (135) soit déplacé depuis la surface (125) de reprise de fluide jusque dans le système de microcavités (130),
assembler (1110) l'élément de couvercle (105), l'élément de fond (115) et le film (122), l'assemblage (1110) s'effectuant en plaçant la découpe de fond (120) face à l'élément de couvercle (105) et le film (122) au niveau de la découpe de fond (120) entre l'élément de couvercle (105) et l'élément de fond (115) et
former (1115) au moins un canal (200) pour fluide entre l'élément de couvercle (105) et l'élément de fond (115) pour guider le fluide (135) sur la surface (125) de reprise de fluide de la découpe de fond (120).
